# EUROPEAN PATENT SPECIFICATION

(11) **EP 1 474 174 B1**
(45) Date of publication and mention of the grant of the patent: **06.05.2009**
(21) Application number: 03703650.6
(22) Date of filing: 14.02.2003
(51) Int. Cl.: A61K 47/44, A61K 9/02, A61K 9/20

(54) **COMPOSITION FOR ORAL OR RECTAL ADMINISTRATION**
ZUSAMMENSETZUNG ZUR ORALEN ODER REKTALEN VERABREICHUNG
COMPOSITION DE PRODUIT A ADMINISTRATION ORALE OU RECTALE

(30) Priority: 15.02.2002 SE 0200475
(43) Date of publication of application: 10.11.2004
(73) Proprietor: DSM IP Assets B.V., 6411 TE Heerlen (NL)
(72) Inventor: HERSLÖF, Bengt, S-11421 Stockholm (SE)
(74) Representative: van Grieken-Plooster, Izabella Johanna
(86) International application number: PCT/SE2003/000251
(87) International publication number: WO 2003/068267

(56) References cited:
- EP-A2- 0 368 247
- WO-A1-02/47663
- DE-A1- 2 951 142
- US-A- 4 699 776
- US-A- 5 082 667
- US-A- 5 716 639
- US-A- 5 989 583
- US-A1- 2002 040 058
- DATABASE BIOSIS [Online] NISHIHATA T. ET AL.: 'An effective formulation for an insulin suppository examination in normal dogs', XP002964966 Database accession no. (PREV198784101370) & INTERNATIONAL JOURNAL OF PHARMACEUTICS (AMSTERDAM) vol. 38, no. 1-3, 1987, pages 83 - 90
- DATABASE BIOSIS [Online] NISHIHATA T. ET AL.: 'Sustained-release of sodium diclofenac from suppository', XP002964967 Database accession no. (PREV19861055696) & INTERNATIONAL JOURNAL OF PHARMACEUTICS (AMSTERDAM) vol. 27, no. 2-3, 1985, pages 245 - 254

## Description

### FIELD OF THE INVENTION

The present invention relates to a pharmaceutical tablet and suppository composition for oral or rectal administration based on lipid carrier materials and to methods for its manufacture and administration.

### BACKGROUND OF THE INVENTION

From a standpoint of patient convenience and production technology the most attractive pharmaceutical form for oral administration of pharmaceutical agents is the tablet but in some cases also rectal administration by a suppository may be advantageous. However far from all pharmaceutical agents are easily formulated as tablets or suppositories. This is true, in particular, to many active principles which are not easily absorbed from the gastrointestinal tract and require, for optimal absorption to be delivered in pharmaceutical carriers comprising lipids which cannot be compounded as tablets or suppositories. Hard or soft shell capsules have to be used instead. However the preferred capsule material gelatin often is not sufficiently inert towards pharmaceutical excipients of this sort and limits the shelf life of the capsule preparation or requires the use of hard gelatin capsules. Hard gelatin capsules are however particularly inconvenient to swallow. In recent years there has also been some concern among consumers in regard of gelatin obtained from animal sources.

On the other hand oral administration of pharmaceutical agents in lipid based carriers contained in capsules undeniably has resulted in improved drug performance in terms of bioavailability. Examples include such compounds as cyclosporin and saquinavir, marketed under the name of Sandimmun Neoral^{®}, Novartis and Invirase^{®}, Roche respectively. Such lipid based carriers are either oily liquids, such as microemulsions, or dispersions, such as emulsions or liposomal preparations, which cannot be easily incorporated into tablets.

Numerous reports describe the use of lipids as tablet excipients in combination with non-lipid constituents. A background of the state of the art in regard of tablet formulations is given in "Modern Pharmaceutics" (Editors G. Banker and C. Rhodes, Marcel Dekker Inc., New York 1996, chapter 10, pp 333 - 394). Most tablets are manufactured by means of powder compression. The pharmaceutical agent(s) is (are) mixed with the excipients to produce a free-flowing powder. Among commonly used excipients are several that can be classified as lipids, for example glycerol triacetate, glycerol behenate, glycerol palmitostearate, zink stearate, magnesium stearate, calcium stearate, stearic acid, hydrogenated vegetable oils, and waxes. Other lipophilic ingredients include paraffins and light mineral oils. Also synthetic lipophilic and amphiphilic ingredients are used, such as polyethylene glycols (PEG), polyoxyethylene monostearates, sodium lauryl sulphate, and sucrose monolaurate.

Most of the aforementioned lipid ingredients act as soluble or insoluble lubricants. They are combined with other types of ingredients, such as fillers (e.g., lactose and starch), binders (e.g., starch mucilage), and disintegrants (e.g., microcrystalline cellulose and cross-linked polyvinylpyrrolidone). Besides their lubricating function lipid ingredients have been used in controlled release formulations.

WO 95/20945 discloses a lipophilic carrier preparation in form of an oily liquid or dispersion having a continuous lipid phase, comprising a non-polar lipid in combination with a polar lipid material, and optionally a polar solvent, polar lipid material being a galactolipid material consisting of at least 50% digalactosyldiacylglycerols, the remainder being other polar lipids.

WO 92/05771 discloses a lipid particle forming matrix containing bioactive material (s) comprising at least two lipid components, one being non-polar and the other amphiphatic and polar. When brought in contact with an aqueous solvent the matrix spontaneously forms discrete lipid particles. The amphiphatic and polar lipid matrix components are said to be bilayer forming and are chosen from phospholipids such as phosphatidylcholine; the non-polar lipids are mono-, di-or triglycerides.

Nishihata et al. Int. J. Pharm. 1987, vol. 38 (1-2) describes a preparation of a solid dispersion of insulin and a triglyceride base containing lecithin which is said to be an effective suppository for lowering blood glucose.

Nishihata et al. Int. J. Pharm. 1985, vol. 27 (2-3) describes the increased solubility of sodium diclofenac in a suppository base in the presence of lecithin.

EP0368247 relates to a matrix preparation produced by dispersing a pharmaceutically active ingredient into a matrix which is solid at ambient temperature and comprised of a fatty acid ester of a polyglycerol. The preparation is said to have stable release-controlling ability.

US5505982 relates to a chocolate that contains a composite of surfactant/cellulose. The composite is made by the process of coprocessing the cellulose with a surfactant. The composite can be used as a bulking agent or functional formulary aid in low-moisture or in fat phase compositions.

WO02/247663 relates to a pharmaceutical composition for oral administration of an active compound showing a bioavailability of 20 % or less. The composition comprises, based on its total weight, from 0.01 % to about 15 % (w/w) of said active compound molecularly dissolved in the composition, from 30 to 80 % (w/w) of an edible lipid matrix and from 1 to 20 % (w/w) of an edible emulsifier, the ratio between the dose weight of the active compound and its solubility in the composition being equal to or greater then 0.6 ml.

### OBJECTS OF THE INVENTION

It is an object of the invention to provide a solid pharmaceutical or food supplement tablet or suppository composition which exploits the advantageous properties of lipids as pharmaceutical carriers in regard of gastroinstestinal uptake and/or sustained release and/or convenience and/or economy.

It is another object of the invention to provide a corresponding carrier composition for incorporation of pharmacologically active or food supplement agents.

It is a further object of the invention to provide processes for making the aforementioned compositions and for incorporating a pharmacologically active agent or food supplement into said carrier composition.

Further objects of the invention will be evident from the following short description of the invention, the description of preferred embodiments, and the appended claims.

### SHORT DESCRIPTION OF THE INVENTION

According to the present invention is disclosed a solid pharmaceutical or food supplement tablet or suppository composition which has a melting point of from 25°C to 50°C or more, preferably from 30°C to 45°C, more preferred from 33°C to 42°C, comprising a continuous lipid phase comprising, preferably consisting of, a polar lipid component, a non-polar lipid component, and one or more agents selected from a pharmacologically active agent and food supplement agent. The polar lipid component consists of one or more polar lipids. The non-polar component consists of one or more non-polar lipids. The one or more polar lipids are membrane lipids, in particular galactolipids. The one or more non-polar lipids are glycerides, i.e. glycerol esters of fatty acids (mono-, di-, and triglycerides). All polar and non-polar lipids of the invention can be sourced from foodstuffs or food grade material. The polar lipids of the invention are amphiphilic with headgroups that are galactose or phosphate esters. The polar lipid component of the invention is combined with the non-polar lipid component in various proportions to allow the controlled incorporation of pharmaceutical including food supplement agents. It is believed that the incorporation mechanism is based on interactions of the polar headgroups and the lipophilic chains of the non-polar component with the compound to be incorporated. Pharmacologically (including food supplementing) efficient compositions for a given pharmacologically active agent or mixture of agents can be experimentally determined by varying the ratio of the polar to non-polar component. To a certain extent the pharmacological or food supplemental efficacy is also influenced by the composition of the polar and non-polar component, respectively.

Preferably the polar component of the composition according to the invention comprises or, more preferred, consists of one or several galactolipids of vegetable origin, such as oat kernels or soybeans. Preferably the non-polar lipid component of the composition according to the invention comprises or, more preferred, consists of one or several glycerides of vegetable origin, such as palmkernel oil, coconut oil, palm oil and cottonseed oil.

It is particularly preferred for the solid pharmaceutical or food supplement tablet or suppository composition of the invention to comprise lipid material of vegetable origin only.

According to the present invention is also disclosed a solid tablet produced from the aforementioned pharmaceutical or food supplement composition, in particular by compression moulding or casting.

According to the present invention is also disclosed a suppository produced from the aforementioned pharmaceutical composition, in particular by compression moulding or casting.

In the pharmaceutical literature lipid continuous phases are described as oily liquids, which need to be administered as oral liquids or enclosed in hard or soft shell capsules. However, such oily liquids are completely outside of the scope of the present invention. Lipid phases are also known in form of dispersions, i.e. dispersed aqueous solvents. Lipid emulsions and liposome preparations are examples of such dispersions which, by definition, are not lipid continuous phases and therefore do not form part of the present invention.

The polar component of the invention can be described as formed of galactolipids which are a class of membrane lipid(s), i.e. the lipid constituents of biological membranes. Membrane lipids contain a polar, hydrophilic, head group and one or more lipophilic hydrocarbon chains. This combination makes the membrane lipid molecules amphipathic and enables them to associate both with water and oils. Such membrane lipids can be classified according to their chemical structure, which is a function of how the polar head group is linked to the lipophilic chains. Sphingolipids (linked by sphingosine) and glycerolipids (linked by glycerol) are the two main groups. Depending on the characteristics of the polar head group sphingolipids and glycerolipids can be further classified as phospholipids, with the head group being a phosphate ester, or as glycolipids, with the head group being a carbohydrate. Depending of the specific nature of the carbohydrate group membrane lipids sometimes are called, for example, galactolipids, which are glycerolipids with galactose in the polar head group. Examples of common membrane lipids are phosphatidylcholine (PC), phosphatidylethanolamine (PE), and digalactosyl-diacylglycerol (DGDG). The membrane lipids can be extracted from, for example, egg yolk (egg lecithin), milk and dairy products, soybeans (soy lecithin), other oil crops, oat kernels, and other cereals and grains. These extracts can be further treated to become, for example, PC from soybeans and galactolipids from oats. Preferred galactolipids useful in the invention are galactolipids from oat kernels (CPL-galactolipid) or from soybeans (soy lecithin or soy-PC). Particularly preferred are partially hydrolysed galactolipids.

Synthetic polar lipids and membrane lipid analogues based on a galactose ester moiety are comprised by the polar lipid component of the invention.

The non-polar lipids of the invention are fatty acid esters of glycerol. These esters include mono-, di-, and triglycerides. Edible oils are triglyceride oils, from which mono-and diglycerides can be derived. Other non- polar lipids of the invention include vegetable and animal oils from various sources, synthetic oils, fatty acids, natural and synthetic glycerides, sterol esters, fatty alcohols. Synthetic non-polar lipids and fatty acid analogues are also comprised by the invention. A description of the area of polar and non-polar lipids is given in "Fatty Acid and Lipid Chemistry" (Frank Gunstone, 1996, Blackie Academic & Professional, Chapman & Hall).

The triglyceride may be selected from palmkernel oil or natural oils with similarly, relatively high solid fat content or melting range. Preferred non-polar lipids include palmkernel oil fractions, obtained by commercial fractionation of palmkernel oil into specific mixtures of triglycerides, e.g. palmkernel stearin, based on the combination of mainly lauric, myristic, and palmitic esters of glycerol. Preferred monoglycerides are selected from edible oil derived monoglycerides, in particular medium chain monoglycerides (chain length C₈ -- C₁₀), derived from coconut oil, and normal chain monoglycerides (chain length C₁₆ - C₁₈) , derived from most vegetable oils.

According to a preferred aspect of the invention the continuous lipid phase may comprise up to 15% by weight, preferably up to 10% by weight, most preferred up to 5% by weight of water and/or an alcohol, including an alkanediol or -triol, such as ethanol, 1,2-propylene glycol, low molecular weight polyethylene glycol, and glycerol. By definition the continuous lipid phase cannot comprise more water or alcohol than is compatible with its property of being continuous.

According to the invention is also disclosed a pharmaceutical or food supplemental or suppository carrier composition consisting of a continuous lipid phase having a melting point of from 25°C to 50°C or more, preferably from 30°C to 45°C, more preferred from 33°C to 42°C, comprising, preferably essentially consisting of, a polar lipid component in combination with a non-polar lipid component.

According to the present invention is furthermore disclosed a process for the production of a pharmaceutical or food supplement tablet composition or suppository composition which has a melting point of from 25°C to 50°C or more, preferably from 30°C to 45°C, more preferred from 33°C to 42°C, comprising a continuous lipid phase comprising, preferably consisting of galactolipids, glyceride esters of fatty acids and a pharmacologically active chemical agent or food supplementing agent, comprising mixing galactolipids with glyceride esters of fatty acids at a first temperature at which at lease one of said components is in a liquid state, thereby obtaining a liquid continuous lipid phase, dissolving one or more of said agents in the liquid continuous lipid phase, cooling the solution thus obtained or aliquots thereof to a second temperature at which it solidifies, said second temperature ranging from 25°C to 50°C or more, preferably from 30°C to 45°C, more preferred from 33°C to 42°C. The cooling may produce a cake if carried out in bulk or a powder if the liquid product is fed to a nozzle, preferably at a temperature slightly above its melting point, and sprayed on, for instance, a cooled metal surface, in particular a polished chromium plated stainless steel surface in form of a band running on rollers. A powderous product may also be obtained by spraying the liquid product into a atmosphere of a temperature below the solidification temperature of the liquid product. The cake may be transformed into powder by, for instance, grinding at a low temperature.

According to a second preferred aspect is disclosed a tablet or suppository of the invention coated with one or several layers of tablet or suppository, respectively, coating excipients, such as to provide the tablet or suppository with an enteric coat and/or a coat physically stabilizing the tablet or suppository at a temperature at or above its melting point, and a corresponding coating process. Particularly preferred is a tablet or suppository of the invention provided with a first or only coat applied by a dry coating process comprising mechanically working a coating powder into the surface of the tablet or suppository at a temperature at which the tablet or suppository is sufficiently soft for the powder particles to adhere and allow them being worked into its surface but not sufficiently soft for substantial deformation, in particular at a temperature from 25°C to 10°C below the melting point of the tablet or suppository. One or more additional layers may be added to the thus coated tablet or suppository by routine pharmaceutical coating processes known in the art. The tablet or suppository of the invention may also be built up around an inert nucleus.

A tablet or suppository according to the invention can be produced from the pharmaceutical or food supplement tablet composition of the invention by compressing the aforementioned powderous product or by moulding or any other suitable process. According to a preferred aspect of the invention the moulding is carried out in a mould covered with an anti-adhering agent or layered with an anti-adhering material, such as amorphous silica, cornstarch and sodium lauryl sulphate, and poly(perfluoro-ethylene), respectively.

The pharmaceutical agent or agents of the invention can be of any type suitable for forming a tablet or suppository composition with the pharmaceutical carrier of the invention, with the proviso that the pharmaceutical agent or agents is soluble in the pharmaceutical carrier and is stable at a temperature above 30°C, preferably above 33°C, most preferred above 40°C, for a time sufficient to incorporate it into the pharmaceutical carrier. In this context "stable" means that no more than 5% by weight of the pharmaceutical agent(s), preferably not more than 2% by weight, most preferred not more than 1% by weight, is degraded or lost during the incorporation process. The term "pharmaceutical agent" comprises any substance that prevents, cures or alleviates an aberrant health state, such as a nutritional defect, in particular vitamin deficiency or a deficiency of essential amino acids, and any substance used for diagnostic purposes which is per-orally administrable. The pharmaceutical agent of the invention can be any of analgesics, anti-inflammatory agents, antihelmintics, antiantiallergic agents, arrhythmic agents, antibacterial agents, anti-coagulants, antidepressants, antidiabetic agents, anti-epileptics, antifungal agents, antigout agents, anti-histamines, antihypertensive agents, antimalarial agents, antimuscarinic agents, antimycobacterial agents, antineoplastic agents, antiprotozoal agents, antithyroid agents, antiviral agents, anxiolytic agents, betaadrenoceptor blocking agents, cardiac inotropic agents, corticosteroids, cough suppressants, diagnostic agents, diuretics, dopaminergics, enzymes, gastro-intestinal agents, hypnotics, hypothalamic hormones, immunological agents, immunosuppressants, lipid regulating agents, mucolytics, muscle relaxants, neuroleptics, nutritional agents, opoid analgesics, parasympathomimetics, pituitary hormones, parathyroid agents, prostaglandins, sedatives, sex hormones, sympathomimetics, thyroid agents, vasodilators, vitamins, and xanthines. The requirements for incorporation of food supplement agents in the tablet or suppository of the invention correspond to those of pharmacologically active agents.

By way of examples it was surprisingly found that the solid pharmaceutical or food supplement tablet or suppository composition of the invention not only can incorporate a wide variety of pharmacologically active agents or food supplement agents of vastly differing chemical structures, but also increases its uptake in the gastrointestinal tract and/or prolongs its efficacy. The present invention thus provides a novel way of improving and widening the use of tablet or suppository compositions for pharmaceutical use including food supplement use.

In the following the invention will be explained in more detail by the following, non-limiting examples.

### DESCRIPTION OF PREFERRED EMBODIMENTS

### Materials. The lipid materials used are listed in Table 1.

If not indicated otherwise all percentages in the description of preferred embodiments are by weight.

**Table 1. Lipid materials**

| Type of lipid | Trade name and source |
|---|---|
| PL-1 | Galactolipids from oats (CPL-Galactolipid; Lipid Technologies Provider AB, Karlshamn, Sweden) |
| PL-2 | PC from soybeans (prepared from soy lecithin Epikuron 135 F; Lucas Meyer GmbH&Co, Hamburg, Germany) |
| MG-1 | Medium chain monoglyceride (Akoline MCM; Karlshamns AB, Karlshamn Sweden) |
| MG-2 | Monoglycerides from edible oils (Dimodan CP; Danisco, Copenhagen, Denmark) |
| TG-1 | Palmkernel stearin (fraction of palmkernel oil ; Karlshamns AB, Karlshamn Sweden) |
| TG-2 | Hydrogenated cotton seed oil (Akofine NF; Karlshamns AB, Karlshamn Sweden) |

### EXAMPLE 1. Exemplary preparation of a tablet by compression of a powderous mixture of lipids (Method A).

### A mixture of the following ingredients (in g) was prepared:

| | |
|---|---|
| Non-polar lipids (hydrogenated triglycerides; Akofine™) | 18,00 |
| Polar lipid material (galactolipids; CPL-Galactolipid™) | 2,00 |
| Vitamin B12 | 0,040 |

The powderous ingredients were blended in a dry mixer. Aliquots (0.50 g) of the homogenous powder were compressed to tablets in a manually operated press (Manesty Machines Ltd, Model no D3). It is also possible to prepare a suppository in this manner by using an appropriate press-form.

### EXAMPLE 2. Exemplary preparation of a tablet by casting molten lipid mixture into a mould (Method B).

### Ingredients (in g):

| | |
|---|---|
| Non-polar lipids (fractionated triglycerides; palmkernel stearin) | 18,00 |
| Polar lipid material (galactolipids; CPL-Galactolipid™) | 2,00 |
| Vitamin B12 | 0,040 |

The ingredients were blended and the mixture melted by heating to a temperature of 60°C and stirred at this temperature for 5 hours when all vitamin B12 had dissolved. Aliquots (0.50 g) of the melted phase were cast in a mould covered with hydrogenated triglyceride (Akofine NF™) powder. The mould was cooled in a freezer and the tablets recovered. A suppository can be prepared in a corresponding manner by using an appropriate mould.

### EXAMPLE 3. Preparation of tablets containing vitamin B12, folic acid, retinyl palmitate or desmopressin (as acetate).

Tablets were prepared according to Method A (as described in Example 1) or Method B (as described in Example 2) with several carrier compositions (Table 1) according to the invention. The 17 preparations thus produced and their relative efficacies are listed in Table 2.

The results demonstrate that the proportions and structure of the lipid phase components affect bioavailability. A range from highly improved (by a factor of 5.3) uptake to highly suppressed uptake, i.e. virtually nil, was observed.

**Table 2. Pharmaceutical/food supplement tablet preparations**

| Prep. no. | Method | Active principle/tablet (0.5 g) | Lipids (% by weight) | | | Efficacy (Reference = 100) |
|---|---|---|---|---|---|---|
| | | Vitamin B12 | Polar (% by weight) | Non-polar lipid (% by weight) | | |
| | | (mg) | | Glyceride I | Glyceride II | |
| 1 | B | 1 | 20 (PL-1) | 5(MG-1) | 75 (TG-1) | 33 |
| 2 | B | 1 | 20 (PL-1) | 10 (MG-1) | 70 (TG-1) | 74 |
| 3 | B | 1 | 20 (PL-1) | 15 (MG-1) | 65 (TG-1) | 529 |
| 4 | B | 1 | 20 (PL-1) | 20 (MG-1) | 60 (TG-1) | 191 |
| 5 | B | 1 | 20 (PL-1) | 30 (MG-1) | 50 (TG-1) | 100 |
| 6 | B | 1 | 45 (PL-1) | 35 (MG-1) | 20 (TG-1) | 355 |
| 7 | B | 1 | 57 (PL-1) | 43 (MG-1) | 0 | 148 |
| 8 | B | 1 | 10 (PL-1) | 0 | 90 (TG-1) | 108 |
| 9 | A | 1 | 10 (PL-1) | 0 | 90 (TG-2) | 6 |
| 10 | B | 1 | 20 (PL-1) | 15 (MG-2) | 65 (TG-1) | 43 |
| 11 | B | 1 | 20 (PL-2) | 15 (MG-1) | 65 (TG-1) | 71 |
| 12 | B | 1 | 20 (PL-2) | 20 (MG-1) | 60 (TG-1) | 0 |
| | | Folic acid (mg) | | | | |
| 13 | B | 5 | 20 (PL-1) | 10 (MG-1) | 70 (TG-1) | 93 |
| 14 | B | 5 | 20 (PL-1) | 15 (MG-1) | 65 (TG-1) | 117 |
| 15 | B | 5 | 20 (PL-1) | 20 (MG-1) | 60 (TG-1) | 56 |
| 16 | B | 5 | 10 (PL-1) | 0 | 90 (TG-1) | 81 |
| 17 | A | 5 | 10 (PL-1) | 0 | 90 (TG-2) | 1 |
| | | Retinyl palmitate (mg) | | | | |
| 18 | B | 33 (50000 IE) | 10 (PL-1) | 0 | 90 (TG-1) | 115 |
| 19 | A | 33 (50000 IE) | 10 (PL-1) | 0 | 90 (TG-2) | 6 |
| | | Desmopressin* (µg) | | | | |
| 20 | B | 50 | 20 (PL-1) | 15 (MG-1) | 65 (TG-1) | ** |

| | | | | | | |
|---|---|---|---|---|---|---|
| * As acetate. ** See Example 5 | | | | | | |

### EXAMPLE 4. Test of tablet preparations in healthy human volunteers.

Tablet preparations of vitamin B12, folic acid, and retinyl palmitate respectively were tested in healthy human volunteers. As reference each person was also given the same dose of active principle in form of a commercial tablet preparation (vitamin B12: Behepan®, Pharmacia; folic acid, Folacin®, Pharmacia; retinyl palmitate: Arovit®, Roche). The observed differences in blood concentration over a given period of time are expressed as percentage of the reference, which was set at 100. Thus a result above 100 for the compositions of the invention indicates an increased plasma concentration of the active principle and thus an increased pharmacological efficacy. These tests were performed with an interval of one week.

The subjects were fasting (intake of water allowed) since 10 p.m. the day before testing. On the testing day the persons arrived at the clinic at 07.00 a.m.. An intravenous catheter was installed in an arm vein for sampling of blood. The tablet was taken at about 7.30 a.m.. A series of blood samples were drawn as outlined in Table 3. In addition

**Table 3. Plasma sampling pattern for vitamin B12, folic acid, and retinyl palmitate**

| *Hours after dosing* | *0,5* | *1* | *2* | *3* | *4* | *6* | *8* |
|---|---|---|---|---|---|---|---|
| *Compound* | | | | | | | |
| Vitamin B12 | | x | x | x | x | x | x |
| Folic acid | x | x | x | x | x | x | x |
| Retinyl palmitate | | x | x | x | x | x | x |

a pre-dosing sample was taken. A standardised lunch was served after the sampling at 4 hours after administration.

The blood samples were treated and analysed in accordance with GCP and validated analytical methods provided by the Laboratory of Clinical Chemistry; Lund University Hospital, Lund, Sweden, and the Laboratory of Clinical Chemistry, Huddinge Hospital, Sweden. Plasma concentrations were plotted against time. The area under the curve obtained from the reference tablet was defined as 100, and the area under the curve (AUC) from the corresponding tablet of the invention was expressed as a percentage of the reference.

The AUC was calculated by the linear trapezoidal rule to the last blood concentration. Except for preparations no. 13, 14, 15 the concentration of the samples taken before administration was regarded as baseline and subtracted from the concentration of sample taken after administration since no samples prior to administration were taken in the latter preparations; the plasma conc. of active principle at start was set to zero. The results are given in Tables 2 and 4-6.
# Indicates the preparation number (see Table 2).

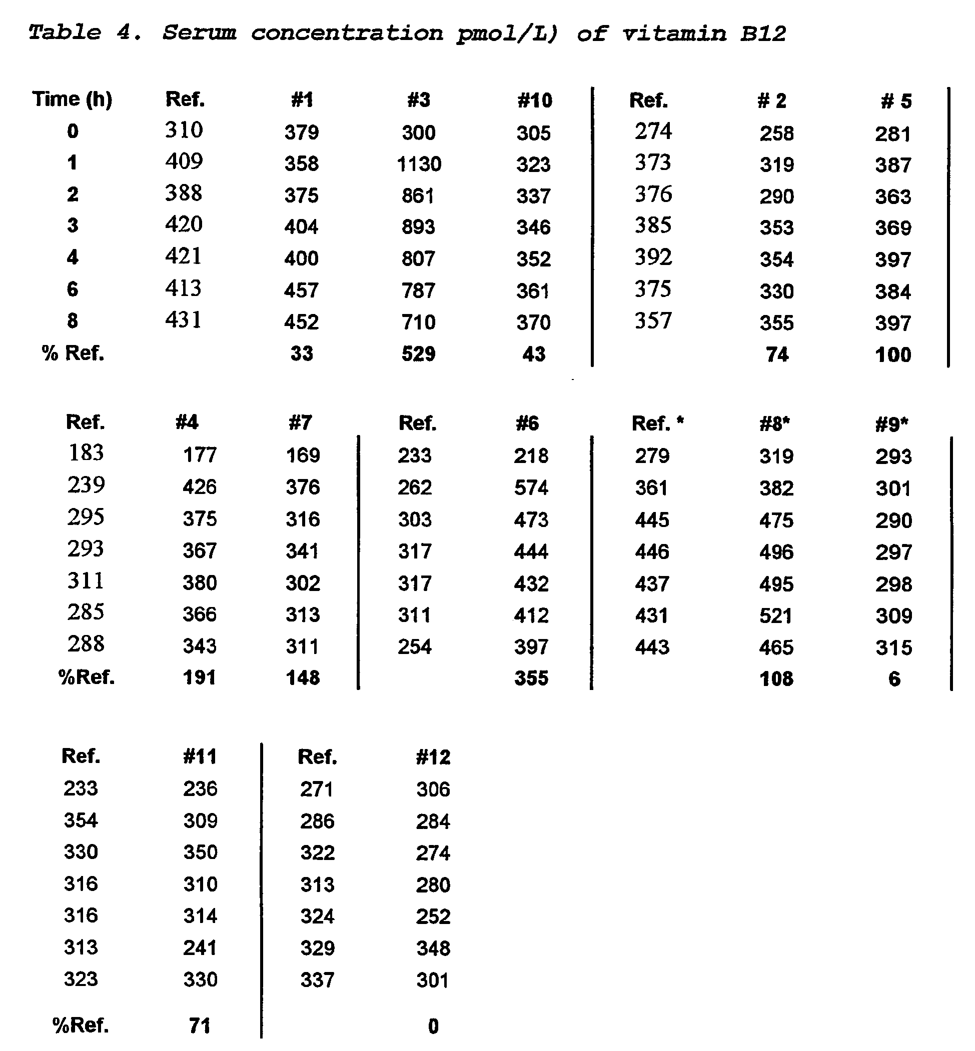

**Table 5. Serum concentration (nmol/Z) of folic acid**

| **Time (h)** | **Ref.** | **#13** | **#15** | **Ref.** | **# 14** |
|---|---|---|---|---|---|
| **0,5** | 25 | 132 | 48 | 9 | 113 |
| **1** | 50 | 399 | 193 | 10 | 193 |
| **2** | 147 | 504 | 204 | 162 | 208 |
| **4** | 525 | 231 | 170 | 273 | 252 |
| **8** | 120 | 63 | 95 | 86 | 97 |
| **% Ref.** | | **93** | **56** | | **117** |

| **Time (h)+A55** | **Ref.** | **#16** | **#17** | | |
|---|---|---|---|---|---|
| 0 | 14,8 | 25 | 23,7 | | |
| 0,5 | 24,8 | 96 | 21,7 | | |
| 1 | 301 | 429 | 20,7 | | |
| 2 | 679 | 477 | 26,2 | | |
| 3 | 453 | 337 | 27,2 | | |
| 4 | 338 | 318 | 29,8 | | |
| 6 | 216 | 152 | 27,6 | | |
| 8 | 117 | 100 | 26 | | |
| **% Ref.** | | **81** | **1** | | |

**Table 6. Serum concentration (micromoi/L) of retinyl palpitate**

| **Time (h)** | **Ref.** | **#18** | **#19** |
|---|---|---|---|
| 0 | 0,02 | 0,04 | 0,03 |
| 1 | 0,03 | 0,07 | 0,03 |
| 2 | 0,04 | 0,48 | 0,04 |
| 3 | 0,38 | 1,19 | 0,05 |
| 4 | 0,79 | 0,92 | 0,05 |
| 6 | 1,83 | 1,52 | 0,13 |
| 8 | 0,53 | 0,64 | 0,12 |
| **% Ref.** | | **115** | **6** |

### EXAMPLE 5. Test of tablet preparations with desmopressin (anti-diuretic) in healthy human volunteers.

This tablet preparation was tested by means of measuring the amount of urine produced over a given period of time according to procedures described in the literature (Hans Vilhardt and Stefan Lundin, Gen. Pharmac. 17 (1986) 481-483). The healthy male volunteers were fasting since 10.00 p.m. the day preceding the test. On the following morning the subject drank an amount of tap water corresponding to 1.5 % of his body weight. Then the urine was collected every 15 minutes. The collected volume was measured and an equal volume of tap water was ingested immediately thereafter. The tablet was taken when the collected volume of urine per period of 15 min exceeded 150 ml. A light breakfast was given one hour after administration of desmopressin, and a light lunch 3 hours later. The liquids consumed to these meals were included in the ingested volumes replacing the collected urine.

The result of the test is expressed as percentage of the accumulated urine production in the tablet of this invention compared to half of the commercial reference tablet containing 100 µg of desmopressin (Minirin®, Ferring) over a period of 11 hours starting 30 min after administration.

The desmopressin composition according to the invention (Preparation 17) increased the anti-diuretic effect of desmopressin 3.5 times in terms of volume of urine produced over a period of 11.5 hours after administration (see, Tables 2 and 7).

### EXAMPLE 6. Preparation of a carbohydrate coated continuous lipid phase tablet.

Vitamin B12 tablets (EXAMPLE 2; 60 g) were fed to a coating cylinder. Simultaneously a powderous mixture of 68% acacia gum, 20% lactose and 12% dextrose (3% by weight of the tablets) was introduced into the cylinder. The mixture was rotated at 30 rpm for 3 hrs at 18°C. The tablets with a smooth surface obtained can be further coated by traditional pharmaceutical coating methods, such as by fluidised bed coating (see, for instance: S C Porter and C H Bruno, Coating of Pharmaceutical Soli d-Dosage Forms, in: Pharmaceutical Dosage Forms, H A Lieberman et al., Eds., 2nd Ed. Vol. 3, p. 77-160, Marcel Dekker, New York and Basel 1990, and literature cited therein).

**Table 7. Urine collected after administration of desmopressin**

| *Min after administration* | *Collected urine (ml)* | |
|---|---|---|
| | Preparation 17 | Reference |
| 30 | 0 | 42 |
| 45 | 0 | 0 |
| 60 | 0 | 0 |
| 75 | 0 | 24 |
| 90 | 0 | 0 |
| 105 | 0 | 0 |
| 120 | 0 | 0 |
| 150 | 0 | 0 |
| 180 | 0 | 50 |
| 210 | 0 | 42 |
| 240 | 140 | 48 |
| 270 | 46 | 60 |
| 300 | 34 | 66 |
| 330 | 32 | 84 |
| 360 | 30 | 120 |
| 390 | 18 | 120 |
| 420 | 20 | 158 |
| 450 | 44 | 206 |
| 480 | 70 | 208 |
| 510 | 40 | 216 |
| 555 | 28 | 322 |
| 600 | 42 | 438 |
| 645 | 98 | 448 |
| 690 | 236 | 432 |
| Accumulated volume | 878 | 3084 |

### EXAMPLE 7. Preparation of a continuous lipid phase tablet containing 1.8 mg of porcine insulin (Method B).

Materials, by weight:
- Non-polar lipids (medium chain monoglycerides; MCMG) 180 parts;
- Non-polar lipids (fractionated triglycerides; palmkernel stearin), 450 parts;
- Polar lipid material (galactolipids; CPL-Galactolipid™), 240 parts;
- Insulin, 1.8 parts;
- 4% Aqueous sodium bicarbonate, 28.2 parts.

Porcine insulin (Sigma, no. I 5523) was dissolved in the sodium bicarbonate solution at 60°C. The monoglyceride was added and the mixture was stirred until a clear liquid had formed. The galactolipids and the palmkernel stearin were subsequently added stepwise at the same temperature. Stirring was continued until clear liquids had formed. On cooling the liquid corresponding to the tablet composition solidified; m.p. 33°C. Aliquots (500 mg) of the molten composition were cast in a mould covered with hydrogenated triglyceride (Akofine NF™) powder. The mould was cooled in a freezer. Upon solidification the solid tablets were recovered by hand.

### EXAMPLE 8. Useful commercially available synthetic lipid materials (examples): Mono- and diglyceride acetates;

mono- and diglyceride citrates; mono- and diglyceride lactates; polyglycerol esters of fatty acids; propyleneglycol esters of fatty acids; sorbitane esters of fatty acids; Sodium and calcium stearoyl lactates; diacetyl tartaric acid esters of mono- and diglycerides; diglycerol esters of fatty acids.

### EXAMPLE 9. Useful commercially available food supplement and other supplementary materials for incorporation into a tablet of the invention (examples):

Amino acids, vitamins and other food supplement agents, in particular lecithin, linseed oil, melatonin, mono-octanoin, peptides, in particular di- to decapeptides, biotin, carnitine, cystine, methionine, isoleucine, leucine, ornithine, lysine acetate, folic acid, vitamin D, cholecalciferol, Vitamin E.

### EXAMPLE 10. Gentamycin sulphate compositions.

The following gentamycin sulphate compositions of the invention ("Gentamycin 2", "Gentamycin 3", "Gentamycin 4") were prepared (Table 8).

**Table 8. Gentamycin sulphate compositions**

| Batch # | Gentamycin sulphate | Composition |
|---|---|---|
| W 21212-N1 "Gentamycin 1" | Gentamycin sulphate batch no. 070K1038; Experimental batch size: 120 mg | Gentamycin sulphate 100% |
| W 20920-N3 "Gentamycin 2" | Gentamycin sulphate batch no. 070K1038; Experimental batch size: 2x4.75g | Gentamycin sulphate 50mg = 1.05%; H₂O 0.5g = 10.5%; Lyso-PC 0.5g = 10.5%; CPL-GL 1.05g = 22.1%; MCMG 1.15g = 24.2%; PK stearin 1.5g = 31.6% |
| W 20920-N2 "Gentamycin 3" | Gentamycin sulphate batch no. 070K1038; Experimental batch size: 2x4.75g | Gentamycin sulphate 50mg = 1.05%; H₂O 0.5g = 10.5%; CPL-GL 1.55g = 32.6%; MCMG 1.15g = 24.2%; P stearin 1.5g = 31.6% |
| W 21106-N2 "Gentamycin 4" | Gentamycin sulphate batch no. 070K1038; Experimental batch size: 4.0g | Gentamycin sulphate 120mg = 3%; H₂O 0.4g = 10%; HGL 1.24g = 31%; MCMG 0.92g = 23%; PK stearin 1.32g = 33% |

| | | |
|---|---|---|
| Abbreviations in Table 8: Lyso-PC: lysophosphatidylcholine; HGL: partially hydrolysed galactolipid (Example 12); MCMG: medium chain monoacylglycerol; CPL-GL: CPL galactolipid; PK stearin: palm kernel oil stearin; P stearin: palm oil stearin. | | |

### EXAMPLE 11. Vancomycin hydrochloride compositions.

The following vancomycin hydrochloride compositions of the invention (Table 9) were prepared by pouring aliquots of the liquid compositions at 50°C into hard gelatin capsules and allowing them to cool and solidify in place.

**Table 9. Vancomycin hydrochloride compositions**

| Batch # | Batch size (g) | Composition | Observations |
|---|---|---|---|
| W 21029-N1 "Vancomycin 1" | 1.0 | Vancomycin hydrochloride: 20mg = 2%; H₂O: 0.15g = 15%; HGL: 0.16g = 16%; CPL-GL: 0.14g = 14%; MCMG: 0.22g = 22%; PK stearin: 0.31g = 31% | |
| W 21107-N1 "Vancomycin 2" | 1.0 | Vancomycin hydrochloride: 20mg = 2%; H₂O: 0.15g = 15%; CPL-GL: 0.31g = 31%; MCMG: 0.23g = 23%; cholesterol 0.1g = 10%; PK stearin: 0.19g = 19% | Substantial improvement over "Vancomycin 1" |
| W 21209-N3 "Vancomycin 3" | 6.0 | Vancomycin hydrochloride: 120mg = 2%; H₂O: 0.9g = 15%; CPL-GL: 1.86g = 31%; MCMG: 1.38g = 23%; cholesterol 0.6g = 10%; PK stearin: 1.14g = 19% | Ca. 85% of water can be removed by evaporation at 60°C; improvement over "Vancomycin 2" |

| | | | |
|---|---|---|---|
| For abbreviations, see Table 8 | | | |

### EXAMPLE 12. Preparation of partially hydrolysed galactolipid (HGL).

Galactolipid (40 g) was dissolved in MeOH (2.0 L) assisted by ultrasound. Aqueous NH₃ (25%; 10 ml) was added. The mixture was shaken at room temperature for 23 hrs; a yellowish green colour and a small amount of a lightly coloured precipitate had formed. The solution was evaporated on a rotary evaporator under reduced pressure. 400 ml of acetone was added to extract free fatty acids. After repeated evaporation at 60°C and standing over night the supernatant was decanted and the residue evaporated and freeze dried after addition of water (300 ml). 31.7 g of a gel containing about 12% of DGMG (digalactosyl-monoacylglycerol), less than 1% of fatty acid methyl esters, and about 2% of digalactosyl-glycerol was formed. The content of DGDG (digalactosyl-diacylglycerol) thus had been reduced to about 40%.

### EXAMPLE 13. Administration of gentamycin.

NZW rabbits were used in all experiments and all tablet/capsules were administered orally. The animals were given four, five or six tablets/capsules followed by water until they had swallowed the tablets/capsules. The animals were deprived of food for about 18 hours before dosing. Blood samples were drawn from the ear veins in sodium citrate vials before dosing and 0.5, 1, 2, 6 and, in some cases, 3 hours after dosing. The blood samples were centrifuged for 10 min at approximately 2000xg to obtain plasma for determination of gentamycin by EMIT 2000 TDM assay on a Hitachi 704 Analyzer (Table 10).

The area under the curve (AUC) was calculated by the linear trapezoidal rule to the last blood concentration. Two different doses (5 or 10 mg/kg bodyweight) were used during the experiments. For comparison of the results of the different formulations the AUC was divided by the respective dose of gentamycin. The obtained plasma concentration for pure gentamycin was set to 1. The obtained plasma concentrations for gentamycin in the three different lipid formulations were then expressed as multiple factors of increasing bioabsorption. Thus, Gentamycin 2 gave 12 times higher absorption than Gentamycin 1 due to incorporation of gentamycin in the lipid matrix.

**Table 10. Plasma concentration of gentamycin (microgram/mL) after oral administration to rabbits**

| Time after administration (hrs) | Gentamycin 1 (in substance) Dose 10 mg/kg; n=3 | Gentamycin 2 (in lipid matrix); dose 5 mg/kg; n=4 | Gentamycin 3 (in lipid matrix); dose 5 mg/kg; n=4 | Gentamycin 4 (in lipid matrix); dose 10 mg/kg; n=3 |
|---|---|---|---|---|
| 0 | 0 | 0 | 0 | 0 |
| 0.5 | 0.01 | 0.07 | 0.09 | 0.10 |
| 1 | 0.01 | 0.22 | 0.04 | 0.07 |
| 2 | 0.02 | 0.11 | 0.07 | 0.08 |
| 3 | - | 0.06 | 0.06 | - |
| 4 | 0.01 | 0.06 | 0.05 | 0.09 |
| 6 | 0.03 | 0.09 | 0.06 | 0.08 |
| AUC | 0.09 | 0.55 | 0.34 | 0.48 |
| AUC adjusted to mg/kg given dose | 0.009 | 0.11 | 0.068 | 0.048 |
| Correlation factor | 1 | 12 | 8 | 5 |

## Claims

1. A solid pharmaceutical or food supplement tablet or suppository composition which has a melting point of 25°C or higher, comprising a continuous lipid component comprising one or more galactolipids, one or more glyceride esters of fatty acids, optionally one or several of water and mono-to trivalent alcohol in an amount of up to 15% by weight of the composition, and one or more agents selected from pharmacologically active agent and food supplement agent.

2. The composition of claim 1, substantially consisting of one or more galactolipids, one or more glyceride esters of fatty acids, and one or more pharmacologically active and/or food supplement agents.

3. The composition of claim 1 or 2, wherein at least one of said galactolipid(s) is partially hydrolysed galactolipid.

4. The composition of claim any one of claims 1-3, wherein said one or more glyceride esters of fatty acids are of vegetable origin.

5. The composition of claim 4, wherein said one or more glyceride esters of fatty acids include triglycerides selected from palmkernel oil fractions obtained by commercial fractionation of palmkernel oil.

6. The composition of claim 4, wherein said one or more glyceride esters of fatty acids include C₈-C₁₀ monoglycerides and/or C₁₆-C₁₈ monoglycerides.

7. The composition of any one of claims 1 and 3-6, comprising water and/or one or more of mono-to trivalent alcohol.

8. The composition of claim 7, wherein the monovalent alcohol is ethanol.

9. The composition of claim 7, wherein the divalent to trivalent alcohol is selected from 1,2-propylene glycol, low molecular weight polyethylene glycol and glycerol.

10. The composition of any one of claims 1-9, wherein said pharmacologically active agent is selected from analgesics, anti-inflammatory agents, antihelmintics, antiallergic agents, arrhythmic agents, antibacterial agents, anticoagulants, antidepressants, antidiabetic agents, antiepileptics, antifungal agents, antigout agents, antihistamines, antihypertensive agents, antimalarial agents, antimuscarinic agents, antimycobacterial agents, antineoplastic agents, antiprotozoal agents, antithyroid agents, antiviral agents, anxiolytic agents, beta-adrenoceptor blocking agents, cardiac inotropic agents, corticosteroids, cough suppressants, diagnostic agents, diuretics, dopaminergics, enzymes, gastro-intestinal agents, hypnotics, hypothalamic hormones, immunological agents, immunosuppresants, lipid regulating agents, mucolytics, muscle relaxants, neuroleptics, nutritional agents, opoid analgesics, parasympathomimetics, pituitary hormones, parathyroid agents, prostaglandins, sedatives, sex hormones, sympathomimetics, thyroid agents, vasodilators, vitamins, and xanthines.

11. The composition of any one of claims 1, 3 and 4 to 10, comprising up to 10% by weight of water.

12. The composition of claim 11, comprising up to5% by weight of water.

13. A process for the production of a pharmaceutical or food supplement tablet or suppository composition which has a melting point of from 25 C and higher, comprising: - mixing one or several galactolipids with one or several glyceride esters of fatty acids at a first temperature at which at least one of said components is in a liquid state, - dissolving, in the liquid continuous lipid phase obtained, one or more pharmacologically active agents, - cooling the solution of said one or more pharmacologically active agents or food supplement agents or portions thereof in the lipid phase to a second temperature at which it solidifies, - forming tablets by carrying out the cooling step with aliquots of the solution or from a bulk product obtained in the cooling step or - forming filled capsules, preferably hard gelatin capsules, by carrying out the cooling step with aliquots of the solution that had been poured into said capsules.

14. The process of claim 13, wherein said first temperature is 25°C and higher.

15. The process of claim 13 or 14, wherein said solution is cooled in bulk, comprising forming a powderous product from said bulk product.

16. The process of claim 13 or 14, wherein said solution is fed to a nozzle and sprayed on a surface or into a cavity having a temperature below the melting point of the liquid, thereby forming a powderous product.

17. A process for the production of a pharmaceutical or food supplement tablet or suppository comprising compressing the powderous product of claim 15 or 16 into a tablet or a suppository.

18. The process of claim 17, comprising covering the punch(es) and/or the die for pressing the tablet or suppository with an anti-adherent prior to compression.

19. The process of claim 18, wherein the anti-adherent is selected from stearic acid or a salt thereof.

20. The process of claim 15, wherein the cooling is carried out by pouring an aliquot of said solution into a mould, thereby forming a tablet or suppository.

21. The process of claim 20, wherein the mould is covered with an anti-adherent prior to pouring.

22. The process of any one of claims 17-21, comprising coating said tablet or suppository with one or several powderous pharmaceutical or food supplement excipients.

23. The process of claim 21, wherein said one or several excipients are mechanically worked into the surface of the tablet so as to form a coating.

24. A pharmaceutical or food supplement tablet or suppository according to claim 1 essentially consisting of a continuous lipid phase, optionally comprising an inert nucleus, wherein the lipid phase may optionally comprise one or several of water and mono-to trivalent alcohol in an amount of up to 15% by weight of the lipid phase, the composition having a melting point of 25°C or higher and comprising one or more galactolipids in combination with one or more glyceride esters of fatty acids, and at least one pharmacologically active agent or food supplement agent.

25. A pharmaceutical or food supplement tablet or suppository according to claim 1 comprising a core which has a melting point of 25°C or higher, the core consisting of a continuous lipid phase and optionally comprising an inert nucleus, the continuous lipid phase comprising one or several galactolipids and one or several glyceride esters of fatty acids, wherein the lipid phase may optionally comprise one or several of water and mono-to trivalent alcohol in an amount of up to 15% by weight of the lipid phase, and one or more pharmacologically active chemical agent or food supplement agent, further comprising a coat consisting of pharmaceutical or food supplement excipients.

26. The tablet or suppository of claim 24 or 25, wherein the coat comprises one or more subcoats consisting of pharmaceutical or food supplement excipients.

27. The tablet or suppository of any one of claims 24 to 26, wherein the one or more pharmacologically active agent is selected from analgesics, anti-inflammatory agents, antihelmintics, antiantiallergic agents, arrhythmic agents, antibacterial agents, anticoagulants, antidepressants, antidiabetic agents, antiepileptics, antifungal agents, antigout agents, antihistamines, antihypertensive agents, antimalarial agents, antimuscarinic agents, antimycobacterial agents, antineoplastic agents, antiprotozoal agents, antithyroid agents, antiviral agents, anxiolytic agents, beta-adrenoceptor blocking agents, cardiac inotropic agents, corticosteroids, cough suppressants, diagnostic agents, diuretics, dopaminergics, enzymes, gastro-intestinal agents, hypnotics, hypothalamic hormones, immunological agents, immunosuppresants, lipid regulating agents, mucolytics, muscle relaxants, neuroleptics, nutritional agents, opoid analgesics, parasympathomimetics, pituitary hormones, parathyroid agents, prostaglandins, sedatives, sex hormones, sympathomimetics, thyroid agents, vasodilators, vitamins, and xanthines.

28. The tablet or suppository of any one of claims 24 to 26, wherein the one or more food supplement agents are selected from amino acids and vitamins.

29. Use of the tablet or suppository of any one of claims 24-27 in the manufacture of a medicament for treating or preventing a condition amenable to treatment or prevention by administration of the pharmacologically active agent present in the said tablet or suppository.

## Patentansprüche

1. Feste pharmazeutische oder Nahrungsergänzungs-Tablettenzusammensetzung oder -Suppositorienzusammensetzung mit einem Schmelzpunkt von 25°C oder mehr, enthaltend eine kontinuierliche Lipidkomponente, die ein oder mehrere Galactolipide, einen oder mehrere Glyceridester von Fettsäuren, gegebenenfalls Wasser und/oder ein- bis dreiwertige Alkohole in einer Menge von bis zu 15 Gew.-% der Zusammensetzung und ein oder mehrere unter pharmakologischen Wirkstoffen und Nahrungsergänzungsmitteln ausgewählte Mittel enthält.

2. Zusammensetzung nach Anspruch 1, im wesentlichen bestehend aus einem oder mehreren Galactolipiden, einem oder mehreren Glyceridestern von Fettsäuren und einem oder mehreren pharmakologischen Wirkstoffen und/oder Nahrungsergänzungsmitteln.

3. Zusammensetzung nach Anspruch 1 oder 2, worin es sich bei mindestens einem der Galactolipide um ein teilhydrolysiertes Galactolipid handelt.

4. Zusammensetzung nach einem der Ansprüche 1-3, worin der eine oder die mehreren Glyceridester von Fettsäuren pflanzlichen Ursprungs sind.

5. Zusammensetzung nach Anspruch 4, worin der eine oder die mehreren Glyceridester von Fettsäuren Triglyceride, die unter durch kommerzielle Fraktionierung von Palmkernöl erhaltenen Palmkernölfraktionen ausgewählt sind, umfassen.

6. Zusammensetzung nach Anspruch 4, worin der eine oder die mehreren Glyceridester von Fettsäuren C₈-C₁₀-Monoglyceride und/oder C₁₆-C₁₈-Monoglyceride umfassen.

7. Zusammensetzung nach einem der Ansprüche 1 und 3-6, enthaltend Wasser und/oder einen oder mehrere ein- bis dreiwertige Alkohole.

8. Zusammensetzung nach Anspruch 7, worin es sich bei dem einwertigen Alkohol um Ethanol handelt.

9. Zusammensetzung nach Anspruch 7, worin der zweibis dreiwertige Alkohol unter 1,2-Propylenglykol, niedermolekularem Polyethylenglykol und Glycerin ausgewählt ist.

10. Zusammensetzung nach einem der Ansprüche 1-9, worin der pharmakologische Wirkstoff unter Analgetika, Antiphlogistika, Antihelmintika, Antiallergika, Arrhythmika, antibakteriellen Mitteln, Antikoagulantien, Antidepressiva, Antidiabetika, Antiepileptika, Antimykotika, Antigichtmitteln, Antihistaminen, Antihypertonika, Antimalariamitteln, Antimuskarinika, gegen Mykobakterien wirkenden Mitteln, Antineoplastika, Antiprotozoika, Antithyroidika, antiviralen Mitteln, Anxiolytika, Beta-Adrenorezeptorenblockern, inotropen Herzmitteln, Corticosteroiden, hustenunterdrückenden Mitteln, Diagnostika, Diuretika, Dopaminergika, Enzymen, Magen-Darm-Mitteln, Hypnotika, Hypothalamushormonen, Immunologika, Immunsuppressiva, lipidregulierenden Mitteln, Mukolytika, Muskelrelaxantien, Neuroleptika, Ernährungsmitteln, Opioid-Analgetika, Parasympathomimetika, Hypophysenhormonen, Nebenschilddrüsenmitteln, Prostaglandinen, Sedativa, Sexualhormonen, Sympathomimetika, Schilddrüsenmitteln, Vasodilatatoren, Vitaminen und Xanthinen ausgewählt ist.

11. Zusammensetzung nach einem der Ansprüche 1, 3 und 4 bis 10, enthaltend bis zu 10 Gew.-% Wasser.

12. Zusammensetzung nach Anspruch 11, enthaltend bis zu 5 Gew.-% Wasser.

13. Verfahren zur Herstellung einer pharmazeutischen oder Nahrungsergänzungs-Tablettenzusammensetzung oder -Suppositorienzusammensetzung mit einem Schmelzpunkt von 25°C oder mehr, bei dem man: - ein oder mehrere Galactolipide mit einem oder mehreren Glyceridestern von Fettsäuren mischt, und zwar bei einer ersten Temperatur, bei der mindestens eine der Komponenten in flüssigem Zustand vorliegt, - in der erhaltenen flüssigen kontinuierlichen Lipidphase einen oder mehrere pharmakologische Wirkstoffe löst, - die Lösung der pharmakologischen Wirkstoffe oder Nahrungsergänzungsmittel oder Portionen davon in der flüssigen Phase auf eine zweite Temperatur, bei der sie fest wird, abkühlt, - durch Durchführung des Abkühlungsschritts mit Aliquots der Lösung oder aus einem im Abkühlungsschritt erhaltenen Bulkprodukt Tabletten formt oder - gefüllte Kapseln, vorzugsweise Hartgelatinekapseln, bildet, indem man den Abkühlungsschritt mit Aliquots der Lösung, die in die Kapseln gegossen worden waren, durchführt.

14. Verfahren nach Anspruch 13, bei dem die erste Temperatur 25°C oder mehr beträgt.

15. Verfahren nach Anspruch 13 oder 14, bei dem man die Lösung in der Masse abkühlt, wobei man aus dem Bulkprodukt ein pulverförmiges Produkt bildet.

16. Verfahren nach Anspruch 13 oder 14, bei dem man die Lösung einer Düse zuführt und auf eine Oberfläche oder in einen Hohlraum mit einer unter dem Schmelzpunkt der Flüssigkeit liegenden Temperatur sprüht und **dadurch** ein pulverförmiges Produkt bildet.

17. Verfahren zur Herstellung einer pharmazeutischen oder Nahrungsergänzungstablette oder eines pharmazeutischen oder Nahrungsergänzungssuppositoriums, bei dem man das pulverförmige Produkt von Anspruch 15 oder 16 zu einer Tablette oder einem Suppositorium verpreßt.

18. Verfahren nach Anspruch 17, bei dem man den Stempel bzw. die Stempel und/oder die Form zum Pressen der Tablette oder des Suppositoriums vor dem Verpressen mit einem Antihaftmittel bedeckt.

19. Verfahren nach Anspruch 18, bei dem man das Antihaftmittel unter Stearinsäure oder einem Salz davon auswählt.

20. Verfahren nach Anspruch 15, bei dem man das Abkühlen durchführt, indem man ein Aliquot der Lösung in eine Form gießt und dadurch eine Tablette oder ein Suppositorium erhält.

21. Verfahren nach Anspruch 20, bei dem man die Form vor dem Gießen mit einem Antihaftmittel bedeckt.

22. Verfahren nach einem der Ansprüche 17-21, bei dem man die Tablette oder das Suppositorium mit einem oder mehreren pulverförmigen pharmazeutischen oder Nahrungsergänzungs-Hilfsstoffen überzieht.

23. Verfahren nach Anspruch 21, bei dem man den einen oder die mehreren Hilfsstoffe mechanisch in die Oberfläche der Tablette einarbeitet, um einen Überzug zu bilden.

24. Pharmazeutische oder Nahrungsergänzungs-Tablette oder pharmazeutisches oder Nahrungsergänzungs-Suppositorium nach Anspruch 1, im wesentlichen bestehend aus einer kontinuierlichen Lipidphase, gegebenenfalls mit einem inerten Nukleus, wobei die Lipidphase gegebenenfalls Wasser und/oder einbis dreiwertige Alkohole in einer Menge von bis zu 15 Gew.-% der Lipidphase enthalten kann, wobei die Zusammensetzung einen Schmelzpunkt von 25°C oder mehr aufweist und ein oder mehrere Galactolipide in Kombination mit einem oder mehreren Glyceridestern von Fettsäuren und mindestens einen pharmakologischen Wirkstoff oder mindestens ein Nahrungsergänzungsmittel enthält.

25. Pharmazeutische oder Nahrungsergänzungs-Tablette oder pharmazeutisches oder Nahrungsergänzungs-Suppositorium nach Anspruch 1, enthaltend einen Kern mit einem Schmelzpunkt von 25°C oder mehr, wobei der Kern aus einer kontinuierlichen Lipidphase besteht und gegebenenfalls einen inerten Nukleus enthält, wobei die kontinuierliche Lipidphase ein oder mehrere Galactolipide und einen oder mehrere Glyceridester von Fettsäuren enthält, wobei die Lipidphase gegebenenfalls Wasser und/oder ein- bis dreiwertige Alkohole in einer Menge von bis zu 15 Gew.-% der Lipidphase enthalten kann, und mindestens einen pharmakologischen Wirkstoff oder mindestens ein Nahrungsergänzungsmittel, ferner enthaltend einen Überzug, der aus pharmazeutischen oder Nahrungsergänzungs-Hilfsstoffen besteht.

26. Tablette oder Suppositorium nach Anspruch 24 oder 25, worin der Überzug einen oder mehrere Unterüberzüge, die aus pharmazeutischen oder Nahrungsergänzungs-Hilfsstoffen bestehen, aufweist.

27. Tablette oder Suppositorium nach einem der Ansprüche 24 bis 26, worin der eine oder die mehreren pharmakologischen Wirkstoffe unter Analgetika, Antiphlogistika, Antihelmintika, Antiallergika, Arrhythmika, antibakteriellen Mitteln, Antikoagulantien, Antidepressiva, Antidiabetika, Antiepileptika, Antimykotika, Antigichtmitteln, Antihistaminen, Antihypertonika, Antimalariamitteln, Antimuskarinika, gegen Mykobakterien wirkenden Mitteln, Antineoplastika, Antiprotozoika, Antithyroidika, antiviralen Mitteln, Anxiolytika, Beta-Adrenorezeptorenblockern, inotropen Herzmitteln, Corticosteroiden, hustenunterdrückenden Mitteln, Diagnostika, Diuretika, Dopaminergika, Enzymen, Magen-Darm-Mitteln, Hypnotika, Hypothalamushormonen, Immunologika, Immunsuppressiva, lipidregulierenden Mitteln, Mukolytika, Muskelrelaxantien, Neuroleptika, Ernährungsmitteln, Opioid-Analgetika, Parasympathomimetika, Hypophysenhormonen, Nebenschilddrüsenmitteln, Prostaglandinen, Sedativa, Sexualhormonen, Sympathomimetika, Schilddrüsenmitteln, Vasodilatatoren, Vitaminen und Xanthinen ausgewählt sind.

28. Tablette oder Suppositorium nach einem der Ansprüche 24 bis 26, worin das eine oder die mehreren Nahrungsergänzungsmittel unter Aminosäuren und Vitaminen ausgewählt sind.

29. Verwendung einer Tablette oder eines Suppositoriums nach einem der Ansprüche 24-27 bei der Herstellung eines Arzneimittels zur Behandlung oder Prävention eines Leidens, das der Behandlung oder Prävention durch Verabreichung des in der Tablette oder dem Suppositorium vorliegenden pharmakologischen Wirkstoffs zugänglich ist.

## Revendications

1. Composition de suppositoire ou de comprimé de complément alimentaire ou pharmaceutique solide ayant un point de fusion de 25°C ou plus, comprenant un composant lipidique continu comprenant un ou plusieurs galactolipides, un ou plusieurs esters de glycéride et d'acides gras, éventuellement un ou plusieurs parmi l'eau et un alcool mono- à trivalent en une quantité allant jusqu'à 15% en poids de la composition, et un ou plusieurs agents choisis parmi un agent pharmacologiquement actif et un agent de complément alimentaire.

2. Composition selon la revendication 1, constituée sensiblement d'un ou plusieurs galactolipides, d'un ou plusieurs esters de glycéride et d'acides gras, et d'un ou plusieurs agents pharmacologiquement actifs et/ou de complément alimentaire.

3. Composition selon la revendication 1 ou 2, dans laquelle au moins un parmi lesdits galactolipides est un galactolipide partiellement hydrolysé.

4. Composition selon l'une quelconque des revendications 1-3, dans laquelle lesdits un ou plusieurs esters de glycéride et d'acides gras sont d'origine végétale.

5. Composition selon la revendication 4, dans laquelle lesdits un ou plusieurs esters de glycéride et d'acides gras comportent des triglycérides choisis parmi des fractions d'huile de palmiste obtenues par fractionnement commercial d'huile de palmiste.

6. Composition selon la revendication 4, dans laquelle lesdits un ou plusieurs esters de glycéride et d'acides gras comportent des monoglycérides en C₈-C₁₀ et/ou des monoglycérides en C₁₆-C₁₈.

7. Composition selon l'une quelconque des revendications 1 et 3-6, comprenant de l'eau et/ou un ou plusieurs alcools mono- à trivalents.

8. Composition selon la revendication 7, dans laquelle l'alcool monovalent est l'éthanol.

9. Composition selon la revendication 7, dans laquelle l'alcool divalent à trivalent est choisi parmi le 1,2-propylène glycol, le polyéthylène glycol de bas poids moléculaire et le glycérol.

10. Composition selon l'une quelconque des revendications 1-9, dans laquelle ledit agent pharmacologiquement actif est choisi parmi les analgésiques, les agents anti-inflammatoires, les antihelminthiques, les agents antiallergiques, les agents anti-arythmisants, les agents antibactériens, les anticoagulants, les antidépresseurs, les agents antidiabétiques, les antiépileptiques, les agents antifongiques, les agents antigoutteux, les antihistaminiques, les agents antihypertenseurs, les agents antipaludéens, les agents antimuscariniques, les agents anti-mycobactériens, les agents antinéoplasiques, les agents anti-protozoaires, les agents antithyroïdiens, les agents antiviraux, les agents anxiolytiques, les bêtabloquants, les agents inotropes cardiaques, les corticostéroïdes, les antitussifs, les agents de diagnostic, les diurétiques, les dopaminergiques, les enzymes, les agents gastro-intestinaux, les hypnotiques, les hormones hypothalamiques, les agents immunologiques, les immunosuppresseurs, les agents de régulation de lipides, les mucolytiques, les myorelaxants, les neuroleptiques, les agents nutritionnels, les analgésiques opioïdes, les parasympathomimétiques, les hormones pituitaires, les agents parathyroïdiens, les prostaglandines, les sédatifs, les hormones sexuelles, les sympathomimétiques, les agents thyroïdiens, les vasodilatateurs, les vitamines et les xanthines.

11. Composition selon l'une quelconque des revendications 1, 3 et 4 à 10, comprenant jusqu'à 10% en poids d'eau.

12. Composition selon la revendication 11, comprenant jusqu'à 5% en poids d'eau.

13. Procédé de production d'une composition de suppositoire ou de comprimé de complément alimentaire ou pharmaceutique ayant un point de fusion de 25°C ou plus, comprenant : - le mélange d'un ou plusieurs galactolipides avec un ou plusieurs esters de glycéride et d'acides gras à une première température à laquelle au moins l'un desdits composants est à l'état liquide, - la solubilisation, dans la phase lipidique continue liquide obtenue, d'un ou plusieurs agents pharmacologiquement actifs, - le refroidissement de la solution desdits un ou plusieurs agents pharmacologiquement actifs ou agents de complément alimentaire ou de portions de ceux-ci dans la phase lipidique jusqu'à une deuxième température à laquelle elle se solidifie, - la formation de comprimés en réalisant l'étape de refroidissement avec des aliquotes de la solution ou à partir d'un produit en vrac obtenu lors de l'étape de refroidissement ou - la formation de capsules chargées, préférablement de capsules de gélatine dure, en réalisant l'étape de refroidissement avec des aliquotes de la solution ayant été versée dans lesdites capsules.

14. Procédé selon la revendication 13, dans laquelle ladite première température est de 25°C ou plus.

15. Procédé selon la revendication 13 ou 14, dans lequel ladite solution est refroidie en vrac, comprenant la formation d'un produit pulvérulent à partir dudit produit en vrac.

16. Procédé selon la revendication 13 ou 14, dans laquelle ladite solution est alimentée dans une buse et pulvérisée sur une surface ou dans une cavité ayant une température inférieure au point de fusion du liquide, formant ainsi un produit pulvérulent.

17. Procédé de production d'un suppositoire ou d'un comprimé de complément alimentaire ou pharmaceutique, comprenant la compression du produit pulvérulent selon la revendication 15 ou 16 en un suppositoire ou un comprimé.

18. Procédé selon la revendication 17, comprenant le revêtement du ou des poinçons et/ou de la filière destinés à presser le suppositoire ou le comprimé, d'un agent anti-adhérent préalablement à la compression.

19. Procédé selon la revendication 18, dans lequel l'agent anti-adhérent est choisi parmi l'acide stéarique ou un sel de celui-ci.

20. Procédé selon la revendication 15, dans lequel le refroidissement est effectué en versant une aliquote de ladite solution dans un moule, formant ainsi un suppositoire ou un comprimé.

21. Procédé selon la revendication 20, dans lequel le moule est revêtu d'un agent anti-adhérent préalablement au coulage.

22. Procédé selon l'une quelconque des revendications 17-21, comprenant l'enrobage dudit suppositoire ou comprimé par un ou plusieurs excipients pour complément alimentaire ou pharmaceutique pulvérulents.

23. Procédé selon la revendication 21, dans lequel lesdits un ou plusieurs excipients sont incorporés mécaniquement dans la surface du comprimé de façon à former un enrobage.

24. Suppositoire ou comprimé de complément alimentaire ou pharmaceutique selon la revendication 1, constitué essentiellement d'une phase lipidique continue, comprenant éventuellement un noyau inerte, dans lequel la phase lipidique peut éventuellement comprendre un ou plusieurs parmi l'eau et un alcool mono- à trivalent en une quantité allant jusqu'à 15% en poids de la phase lipidique, la composition ayant un point de fusion de 25°C ou plus et comprenant un ou plusieurs galactolipides en association avec un ou plusieurs esters de glycéride et d'acides gras, et au moins un agent pharmacologiquement actif ou agent de complément alimentaire.

25. Suppositoire ou comprimé de complément alimentaire ou pharmaceutique selon la revendication 1, comprenant un coeur ayant un point de fusion de 25°C ou plus, le coeur étant constitué d'une phase lipidique continue, et comprenant éventuellement un noyau inerte, la phase lipidique continue comprenant un ou plusieurs galactolipides et un ou plusieurs esters de glycéride et d'acides gras, où la phase lipidique peut éventuellement comprendre un ou plusieurs parmi l'eau et un alcool mono- à trivalent en une quantité allant jusqu'à 15% en poids de la phase lipidique, et un ou plusieurs agents chimiques pharmacologiquement actifs ou agents de complément alimentaire, comprenant en outre un enrobage constitué d'excipients de complément alimentaire ou pharmaceutique.

26. Suppositoire ou comprimé selon la revendication 24 ou 25, dans lequel l'enrobage comprend un ou plusieurs sous-enrobages constitués d'excipients de complément alimentaire ou pharmaceutique.

27. Suppositoire ou comprimé selon l'une quelconque des revendications 24 à 26, dans lequel lesdits un ou plusieurs agents pharmacologiquement actifs sont choisis parmi les analgésiques, les agents anti-inflammatoires, les antihelminthiques, les agents antiallergiques, les agents anti-arythmisants, les agents antibactériens, les anticoagulants, les antidépresseurs, les agents antidiabétiques, les antiépileptiques, les agents antifongiques, les agents antigoutteux, les antihistaminiques, les agents antihypertenseurs, les agents antipaludéens, les agents antimuscariniques, les agents anti-mycobactériens, les agents antinéoplasiques, les agents anti-protozoaires, les agents antithyroïdiens, les agents antiviraux, les agents anxiolytiques, les bêtabloquants, les agents inotropes cardiaques, les corticostéroïdes, les antitussifs, les agents de diagnostic, les diurétiques, les dopaminergiques, les enzymes, les agents gastro-intestinaux, les hypnotiques, les hormones hypothalamiques, les agents immunologiques, les immunosuppresseurs, les agents de régulation de lipides, les mucolytiques, les myorelaxants, les neuroleptiques, les agents nutritionnels, les analgésiques opioïdes, les parasympathomimétiques, les hormones pituitaires, les agents parathyroïdiens, les prostaglandines, les sédatifs, les hormones sexuelles, les sympathomimétiques, les agents thyroïdiens, les vasodilatateurs, les vitamines et les xanthines.

28. Suppositoire ou comprimé selon l'une quelconque des revendications 24 à 26, dans lequel lesdits un ou plusieurs agents de complément alimentaire sont choisis parmi les acides aminés et les vitamines.

29. Utilisation du suppositoire ou du comprimé selon l'une quelconque des revendications 24-27, dans la fabrication d'un médicament destiné au traitement ou à la prévention d'une condition pouvant être disposée à un traitement ou une prévention par l'administration de l'agent pharmacologiquement actif présent dans ledit suppositoire ou comprimé.
